## Europäisches Patentamt

## European Patent Office

## Office européen des brevets

(11) Veröffentlichungsnummer: **0 107 769**
**B1**

## EUROPÄISCHE PATENTSCHRIFT

(45) Veröffentlichungstag der Patentschrift:
**10.12.86**

(51) Int. Cl.⁴: **G 01 N 33/20,** G 01 N 31/22

(21) Anmeldenummer: **83108918.0**

(22) Anmeldetag: **09.09.83**

(54) **Flüssigkeit und Verfahren zum Nachweis eines Chromatbelages auf einer Aluminiumfläche.**

(30) Priorität: **29.10.82 US 437702**

(43) Veröffentlichungstag der Anmeldung:
**09.05.84 Patentblatt 84/19**

(45) Bekanntmachung des Hinweises auf die Patenterteilung:
**10.12.86 Patentblatt 86/50**

(84) Benannte Vertragsstaaten:
**CH DE FR GB IT LI SE**

(56) Entgegenhaltungen:
**US - A - 4 244 693**

**Patent Abstracts of Japan, Band 4, Nr. 95, 9. Juli 1980,
Seite 50P18**

(73) Patentinhaber: **DEERE & COMPANY, 1 John Deere Road,
Moline Illinois 61265 (US)**

(72) Erfinder: **Wilson, Lance Kyle, 1106 Bertch Avenue,
Waterloo Iowa 50702 (US)**

(74) Vertreter: **Gramm, Werner, Prof. Dipl.-Ing. et al,
Patentanwälte Gramm + Lins
Theodor-Heuss-Strasse 2, D-3300 Braunschweig (DE)**

## Beschreibung

Die Erfindung betrifft ein Verfahren zum Nachweis eines Chromatbelages auf einer Aluminiumfläche.

Aus Gründen der Gewichtsersparnis bestehen heute viele Maschinenteile aus Aluminium. Bei der Verwendung von Aluminium ist es erforderlich, die Oberfläche mit Chromsäure zu behandeln, um Oxyde zu zu entfernen und so die Haftung eines Anstriches zu verbessern. Bei einer derartigen Behandlung erhält das Aluminiumteil ein sehr schwach hellgoldenes Aussehen. Jedoch ist es in den heutigen modernen Fabriken, in denen Natriumdampflampen Verwendung finden, manchmal sehr schwierig für eine Person, das Vorhandensein eines Chromatbelages durch blosse Inaugenscheinnahme zu erkennen. Bisher ist das Vorhandensein eines derartigen Belages nur durch eine ausserordentlich zeitaufwendige Analyse der atomaren Absorption positiv festzustellen.

Der Erfindung liegt somit die Aufgabe zugrunde, ein Verfahren zu entwickeln, mit dem sich in einfacher und zuverlässsiger Weise ein Chromatbelag auf mit einem Farbanstrich zu versehenden Aluminiumteilen nachweisen lässt.

Diese Aufgabe wird gemäss der Erfindung dadurch gelöst, dass auf die Aluminiumfläche eine Flüssigkeit aufgebracht wird, die eine Rote-Rübe-Pigmentierung aufweist und aus einer Mischung aus zumindest einem Teil einer Wasserlösung von zumindest 0,5 g Kaliumpermanganat (MnO$_4$) pro 100 ml Wasser und zumindest einem Teil einer konzentrierten Natriumhydroxydlösung besteht.

Die verwendete Flüssigkeit reagiert auf der Aluminiumfläche und verändert dabei ihre Farbe, und zwar von der Rote-Rübe-Pigmentierung zu einer hellen blau-grünen Pigmentierung, falls Chromatbelag vorhanden ist, bzw. von der Rote-Rübe-Pigmentierung zu einer beigen Farbe, falls kein Chromatbelag vorhanden ist.

Diese Flüssigkeit lässt sich leicht herstellen, vor allem aber einfach, preiswert und schnell anwenden, wobei die starke Farbänderung eine zuverlässige Aussage über das Vorhandensein eines Chromatbelages sicherstellt.

Die Konzentration des übermangansauren Kali beträgt vorzugsweise 0,5 bis 1,0 G/V% (0,5 bis 1,0 g KMnO$_4$ pro 100 ml Wasser), wobei das konzentrierte Natriumhydroxyd vorzugsweise in einer wässrigen Lösung gesättigt ist.

Die Flüssigkeit kann grundsätzlich zu gleichen Teilen aus dem übermangansauren Kali und der konzentrierten Natriumhydroxydlösung bestehen. Die besten Resultate erhält man aber dann, wenn der Anteil der konzentrierten Natriumhydroxydlösung den des übermangansauren Kali übersteigt. Vorzugsweise können zumindest zwei Teile übermangansaures Kali in einer Konzentration von 0,5 bis 1,0 G/V% und zumindest drei Teile konzentrierte Natriumhydroxydlösung vorgesehen werden. Ein höherer Anteil an Natriumhydroxyd führt zu einer Beschleunigung der Reaktion.

Von dieser Flüssigkeit werden einige Tropfen auf die Oberfläche eines Aluminiumteiles gebracht, wobei die Flüssigkeit vor ihrem Auftrag eine Rote-Rübe-Pigmentierung aufweist, da das übermangansaure Kali eine Rote-Rübe-Farbe aufweist, während konzentriertes Natriumhydroxyd farblos ist und bei seiner Vermischung die rote Farbe des übermangansauren Kali nicht verändert.

Nach dem Auftrag der Flüssigkeit auf die Oberfläche eines Aluminiumteiles verändert sich die Farbe der Flüssigkeit in ein stumpfes Braun oder Beige. Konzentriertes Natriumhydroxyd ist sehr ätzend und wirkt als Katalysator, der die fortschreitende Reaktion zwischen dem Aluminium und dem übermangansauren Kali ermöglicht.

Die beiden vorstehend genannten Bestandteile der Flüssigkeit können vor ihrem Auftrag auf die Aluminiumfläche miteinander vermischt werden, um erst anschliessend die so erhaltene Flüssigkeit auf die Aluminiumfläche aufzutragen. Durch Versuche wurde es jedoch als ebenfalls möglich nachgewiesen, zuerst 0,1 ml übermangansaures Kali mit einer Konzentration von zumindest 0,5 G/V% auf eine Aluminiumoberfläche aufzutragen und erst anschliessend 0,15 ml konzentriertes Natriumhydroxyd auf das übermangansaure Kali zu geben. Innerhalb von etwa 30 bis 60 Sekunden reagiert die Mischung mit der Aluminiumoberfläche und die oben erläuterte Farbänderung tritt ein. Ebenso wäre es aber möglich, zuerst das konzentrierte Natriumhydroxyd und erst anschliessend das übermangansaure Kali aufzutragen.

Beispiel

Das nachfolgende Verfahren wurde angewendet zum Nachweis eines vorhandenen oder nicht vorhandenen Chromatbelages auf zahlreichen Aluminiumteilen, von denen nur einige einen derartigen Belag aufwiesen, die sich jedoch visuell nicht von den anderen Teilen unterscheiden liessen. Zuerst wurde etwa 0,1 ml übermangansaures Kali in einer Konzentration zwischen 0,5 und 1,0 G/V% auf einen kleinen Oberflächenbereich der Aluminiumteile aufgetragen. Unmittelbar anschliessend wurde 0,15 ml konzentriertes Natriumhydroxyd gesättigt in einer wässrigen Lösung, auf das übermangansaure Kali aufgebracht. Die so kombinierte Flüssigkeit wies eine Rote-Rübe-Farbe auf. Ungefähr 30 bis 60 Sekunden nach dem Auftrag auf die mit einem Chromatbelag versehenen Aluminiumoberflächen veränderte sich die Rote-Rübe-Farbe in ein helles Blau-Grün. Entsprechende Teste auf Aluminiumteilen ohne Chromatbelag führten zu einer Farbveränderung von der Rote-Rübe-Farbe in ein Beige oder stumpfes Braun. Diese Farbe zeigte an, dass die Flüssigkeit mit dem Aluminium und seinen Bestandteilen reagiert hatte.

**Patentansprüche**

1. Verfahren zum Nachweis eines Chromatbelages auf einer Aluminiumfläche, dadurch gekennzeichnet, dass auf die Aluminiumfläche eine Flüs-

nate ($KMnO_4$) and one to three parts of NaOH.